# FASCICULE DE BREVET EUROPEEN

(11) **EP 2 775 940 B1**
(45) Date de publication et mention de la délivrance du brevet: **18.11.2015**
(21) Numéro de dépôt: 12794436.1
(22) Date de dépôt: 06.11.2012
(51) Int. Cl.: A61B 17/064

(54) **AGRAFE POUR OSTÉOSYNTHÈSE**
OSTEOSYNTHESEKLAMMER
OSTEOSYNTHESIS CLIP

(30) Priorité: 07.11.2011 FR 1160097
(43) Date de publication de la demande: 17.09.2014
(73) Titulaire: Synchro Medical, 68920 Wettolsheim Les Erlen (FR)
(72) Inventeur: AVEROUS, Christophe, 67206 Mittelhausbergen (FR); CERMOLACCE, Christophe, 13008 Marseille (FR); DETERME, Patrice, 31400 Toulouse (FR); GUILLO, Stéphane, 33000 Bordeaux (FR); ROCHER, Hubert, 33600 Pessac (FR); ROY, Christophe, 26300 Chatuzange le Goubet (FR); ROUSSOULY, Jean-Charles, 69100 Villeurbanne (FR); FIQUET, Arnaud, 69300 Caluire (FR); SEUTIN, Bertrand, 26160 La Batie Rolland (FR); VAZ, Gualter, 69007 Lyon (FR)
(74) Mandataire: Delorme, Nicolas
(86) Numéro de dépôt international: PCT/FR2012/052550
(87) Numéro de publication internationale: WO 2013/068682

(56) Documents cités:
- EP-A1- 0 852 128
- EP-A2- 1 504 723
- WO-A1-89/04146
- US-A1- 2008 077 144
- US-A1- 2008 319 443
- US-A1- 2010 042 160

## Description

La présente invention porte sur une agrafe pour ostéosynthèse, par exemple pour réaliser des ostéotomies, des arthrodèses entre deux fragments osseux, ou encore pour fixer des tissus mous à l'os.

De nombreuses agrafes chirurgicales pour la réparation osseuse existent. Certaines de ces agrafes présentent une forme globale en U, les extrémités distales des barres verticales du U étant destinées à être insérées dans deux fragments osseux différents, en vue de les rapprocher par exemple.

Dans la présente demande, l'extrémité distale d'un dispositif, tel qu'un implant ou une agrafe, doit être comprise comme signifiant l'extrémité la plus éloignée de la main du chirurgien et l'extrémité proximale doit être comprise comme signifiant l'extrémité la plus proche de la main du chirurgien. De même, dans cette demande, la « direction distale » doit être comprise comme signifiant la direction d'impaction de l'agrafe et la « direction proximale » doit être comprise comme signifiant la direction opposée à la direction d'impaction.

En général, préalablement à l'installation d'une agrafe en U, on perce deux trous, un dans chacun des deux fragments osseux destinés à être rapprochés, puis on impacte l'agrafe, chaque barre verticale dans un trou. Une fois l'agrafe impactée, il est très difficile de pouvoir agir sur la compression exercée par les deux barres verticales : ainsi, si cette compression est trop faible, le rapprochement des deux fragments osseux ne sera pas optimal. De même, si cette compression est trop élevée, il n'existe pas de moyen de relâcher cette pression.

Il existe donc le besoin d'une agrafe en forme globale de U permettant l'ajustement de la compression exercée par les deux barres verticales du U, par exemple sur deux fragments d'os à rapprocher, en particulier une fois que l'agrafe a été impactée, c'est-à-dire une fois qu'elle a été pré-installée avec les deux barres verticales du U déjà insérées dans les fragments osseux.

La présente invention vise à remédier à ce besoin en proposant une agrafe en forme globale de U dont la compression exercée par les deux barres verticales du U peut être augmentée ou réduite après impaction de l'agrafe.

La présente invention porte sur une agrafe pour ostéosynthèse en forme globale de U, comprenant :
- au moins deux barres d'ancrage correspondant aux deux barres verticales du U, chaque barre d'ancrage comprenant une extrémité distale, destinée à être insérée dans un élément osseux, et une extrémité proximale, et
- au moins une barre de liaison, correspondant à la barre horizontale du U, reliant entre elles les extrémités proximales respectives des deux barres d'ancrage, ladite barre de liaison comprenant au moins une ouverture longitudinale la séparant en deux parois latérales opposées reliées entre elles à leurs extrémités, déformables dans le plan P contenant la barre de liaison et perpendiculaire au plan contenant l'agrafe en forme de U, de telle sorte que l'ouverture est apte à adopter une configuration fermée, dans laquelle les deux barres d'ancrage sont sensiblement parallèles entre elles et lesdites deux parois latérales sont rapprochées l'une de l'autre de telle sorte que la distance séparant les deux extrémités proximales des deux barres d'ancrage a une valeur L1, et au moins une configuration ouverte, dans laquelle les régions centrales desdites deux parois latérales sont éloignées l'une de l'autre de part et d'autre de ladite ouverture, et la distance séparant les deux extrémités proximales des deux barres d'ancrage a une valeur L2 strictement inférieure à L1,
   caractérisée en ce que
   ladite agrafe comprend en outre une pièce de compression présentant au moins une portion dite portion active, dont la section dans un plan A de ladite pièce présente une forme globalement ovale, ladite pièce étant logée au sein de ladite ouverture avec son plan A confondu avec ledit plan P et au moins une partie de la face externe de sa portion active en contact avec au moins une partie des faces internes desdites deux parois latérales de la barre de liaison, ladite pièce étant apte à subir une rotation autour d'un axe B passant par le centre de ladite forme ovale de ladite portion active et perpendiculaire audit plan P, causant ainsi le passage de ladite ouverture de sa configuration fermée à au moins une dite configuration ouverte, et vice versa.

Ainsi, dans l'agrafe selon l'invention, la distance entre les deux extrémités proximales des deux barres d'ancrage peut varier entre L1 et L2 grâce à la rotation de la pièce de compression logée dans l'ouverture pratiquée dans la barre de liaison. En effet, cette pièce de compression présentant dans son plan A une forme globalement ovale, sa rotation dans le plan P déforme les parois latérales en les obligeant à s'écarter l'une de l'autre, provoquant ainsi une variation de la largeur de l'ouverture pratiquée dans la barre de liaison et le rapprochement l'une de l'autre des extrémités proximales des deux barres d'ancrage.

Par « globalement ovale », on entend, au sens de la présente demande, une forme ovale, elliptique, oblongue ou encore ovoïde, ou plus généralement toute forme autre qu'un disque ou cercle inscrite dans un ovale, présentant un axe de révolution mais dont le rayon n'est pas constant.

Ladite pièce, du fait qu'elle est logée dans ladite ouverture, reste accessible par l'utilisateur, par exemple un chirurgien, même une fois que les deux barres d'ancrage sont insérées dans les éléments osseux, ou autres organes, qu'elles sont destinées à rapprocher. Ainsi, le chirurgien peut, au besoin au moyen d'un outil adapté, imprimer à ladite pièce un mouvement de rotation pour faire varier la distance entre les deux extrémités proximales des deux barres d'ancrage.

En faisant varier la distance entre les deux extrémités proximales des deux barres d'ancrage, on fait également varier la distance séparant les deux barres d'ancrage et on fait ainsi varier la compression exercée par les deux barres d'ancrage sur les éléments osseux ou organes dans lesquels elles sont insérées par leurs extrémités distales.

L'agrafe selon l'invention permet le maintien post-opératoire des éléments osseux dans une position physiologique normale et une compression pendant le temps de la consolidation osseuse.

Par exemple, une ou plusieurs agrafes selon l'invention peuvent être utilisées dans le traitement de l'Hallux Rigidus : après avivement des surfaces articulaires, ces dernières sont maintenues en contact étroit par une ou plusieurs agrafes selon l'invention, pendant le temps de la consolidation.

L'agrafe selon l'invention peut ainsi être utilisée pour le rapprochement de fragments d'os du pied ou de la main ou autre ou encore pour l'arthrodèse d'une articulation. L'agrafe selon l'invention peut également être utilisée pour le rapprochement de deux vertèbres adjacentes.

Dans une forme de réalisation, l'agrafe selon l'invention comprend des moyens de guidage de la rotation de ladite pièce dans ledit plan P. Par exemple, les moyens de guidage comprennent au moins une rainure et un bourrelet aptes à s'engager l'un dans l'autre pour guider la rotation de ladite pièce dans ledit plan P, l'un de ladite rainure et dudit bourrelet étant situé sur les faces internes desdites parois latérales, l'autre de ladite rainure et dudit bourrelet étant situé sur ladite face externe de ladite portion active de ladite pièce. Par exemple, la face interne de chaque paroi latérale peut comprendre une rainure, située dans le plan P, et la face externe de la portion active de la pièce de compression comporte un bourrelet périphérique, également situé dans le plan P, de telle sorte que le bourrelet est engagé dans la rainure de chacune des deux parois latérales : ainsi, lorsque la pièce de compression tourne autour de son axe B, le bourrelet reste engagé dans les deux rainures, assurant que la portion active de la pièce de compression reste bien dans le plan P, provoquant ainsi l'écartement ou le rapprochement des deux parois latérales. Dans une autre forme de réalisation, la face interne de chaque paroi latérale peut comprendre un bourrelet, situé dans le plan P, et la face externe de la portion active de la pièce de compression comporte une rainure périphérique, également située dans le plan P, de telle sorte que la rainure périphérique est engagée dans le bourrelet de chacune des deux parois latérales.

Dans une forme de réalisation, l'agrafe selon l'invention comprend des moyens de blocage de ladite pièce à différents angles de rotation. Par exemple, les moyens de blocage comprennent une pluralité de reliefs répartis selon la périphérie de ladite portion active, aptes à s'engager successivement dans un ou plusieurs reliefs complémentaires situés sur les faces internes desdites parois latérales, l'engagement d'un ou des reliefs dans le ou les reliefs complémentaires bloquant ladite pièce en rotation à un angle de rotation déterminé. La portion active de la pièce de compression ayant une forme globalement ovale, cette forme de réalisation permet de choisir une largeur préférée pour l'ouverture et donc d'ajuster la distance préférée entre les extrémités proximales des deux barres d'ancrage, et par conséquent la force de la compression exercée par ces deux barres d'ancrage sur les éléments osseux ou autres organes dans lesquels elles sont insérées. La pluralité de reliefs répartis sur la périphérie de la portion active permet en effet d'avoir le choix entre plusieurs largeurs possibles pour l'ouverture, que l'on peut bloquer par engagement d'un relief dans le relief complémentaire.

Dans une forme de réalisation, l'agrafe selon l'invention comprend des moyens de désalignement desdites barres d'ancrage, agencés pour provoquer alternativement l'écartement ou le rapprochement l'une de l'autre des régions distales respectives desdites deux barres d'ancrage, dans une configuration ouverte de ladite ouverture. Par exemple, lesdits moyens de désalignement comprennent une fente longitudinale ménagée dans chaque paroi latérale et séparant ladite paroi en deux tronçons longitudinaux, un tronçon proximal et un tronçon distal, reliés entre eux par leurs extrémités, chacun desdits tronçons proximal et distal étant déformable dans le plan P indépendamment de l'autre tronçon.

Ainsi, dans une forme de réalisation de l'invention, ladite partie de ladite face externe de ladite portion active de ladite pièce étant en contact avec les faces internes desdits tronçons proximaux desdites parois latérales seulement, lesdites régions distales desdites deux barres d'ancrage s'écartent l'une de l'autre quand ladite ouverture est dans une configuration ouverte.

Dans une autre forme de réalisation de l'invention, ladite partie de ladite face externe de ladite portion active de ladite pièce étant en contact avec les faces internes desdits tronçons distaux desdites parois latérales seulement, lesdites régions distales desdites deux barres d'ancrage se rapprochent l'une de l'autre quand ladite ouverture est dans une configuration ouverte. Cette forme de réalisation permet de renforcer encore la compression exercée par les barres d'ancrages sur les éléments osseux, ou autres organes, en vue de les rapprocher.

Dans une forme de réalisation de l'invention, les régions distales desdites deux barres d'ancrage sont munies de crans d'accroche destinés à renforcer l'ancrage desdites barres d'ancrage dans les éléments osseux.

Dans une forme de réalisation de l'invention, la face proximale de ladite pièce comporte une surface apte à coopérer avec un outil afin de transmettre un mouvement de rotation à ladite pièce. Par exemple, ladite surface présente un évidement ayant un profil hexagonal, ledit évidement étant apte à accueillir une clé de profil correspondant permettant de faire tourner la pièce de compression au sein de l'ouverture dans laquelle elle est logée.

Les avantages de la présente invention vont ressortir plus clairement de la description qui suit et des dessins annexés dans lesquels :
La figure 1 est une vue en perspective éclatée d'une agrafe selon l'invention,
La figure 2 est une vue en perspective de l'agrafe de la figure 1 avec la pièce de compression logée dans l'ouverture,
La figure 3 est une vue de dessus de l'agrafe de la figure 2 dans la configuration fermée de l'ouverture,
La figure 4 est une vue de dessus de l'agrafe de la figure 2 dans une configuration ouverte de l'ouverture,
La figure 5 est une vue en perspective éclatée d'une première variante de l'agrafe selon l'invention,
La figure 6 est une vue de dessus d'une deuxième variante de l'agrafe selon l'invention, dans la configuration fermée de l'ouverture,
La figure 7 est une vue de dessus de l'agrafe de la figure 6 dans une configuration ouverte bloquée de l'ouverture,
La figure 8 est une vue en coupe de l'agrafe de la figure 1 une fois implantée, dont l'ouverture est en configuration ouverte, rapprochant deux éléments osseux,
La figure 9A est une vue en perspective et en coupe d'une pièce de compression utilisable avec la partie en U d'une agrafe similaire à celle de la figure 5, en vue d'obtenir un rapprochement des régions distales des barres d'ancrage en configuration ouverte de l'ouverture,
La figure 9B est une vue en perspective de la partie en U de l'agrafe utilisable avec la pièce de compression de la figure 9A, en configuration ouverte de l'ouverture ; cependant, sur la Figure 9B, pour des raisons de clarté, la pièce de compression n'est pas représentée,
La figure 10A est une vue en perspective et en coupe d'une pièce de compression utilisable avec la partie en U d'une agrafe similaire à celle de la figure 5, en vue d'obtenir un écartement des régions distales des barres d'ancrage en configuration ouverte de l'ouverture,
La figure 10B est une vue en perspective de la partie en U de l'agrafe utilisable avec la pièce de compression de la figure 10A, en configuration ouverte de l'ouverture ; cependant, sur la Figure 10B, pour des raisons de clarté, la pièce de compression n'est pas représentée.

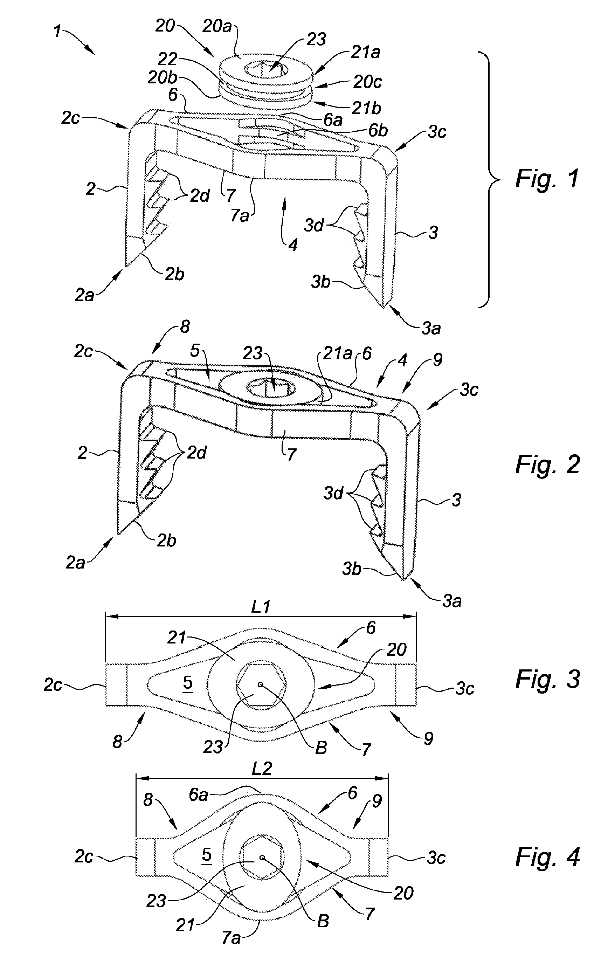

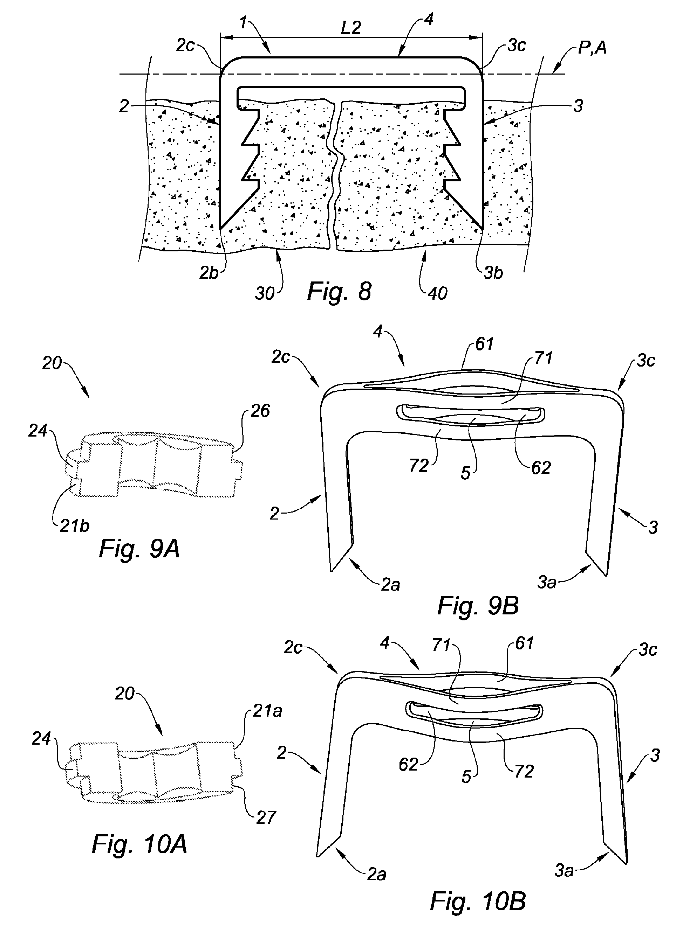

En référence aux figures 1 et 2 est représentée une agrafe 1 selon l'invention, par exemple pour le rapprochement de deux fragments osseux (voir figure 8). Les fragments osseux peuvent être des fragments osseux du pied ou de la main, ou encore deux vertèbres adjacentes à rapprocher. L'agrafe 1 a une forme globale de U et est globalement comprise dans le plan du U, aussi appelé plan de l'agrafe 1.

L'agrafe 1 comprend deux barres d'ancrages (2, 3) correspondant aux barres verticales du U. Chaque barre d'ancrage (2, 3) comprend une extrémité distale (2a, 3a) destinée à être insérée dans un fragment osseux. Comme il apparaît sur ces figures, les extrémités distales (2a, 3a) des barres d'ancrages (2,3) présentent une partie pointue (2b, 3b) pour faciliter la pénétration des barres d'ancrages (2, 3) dans les fragments osseux. Chaque barre d'ancrage (2, 3) présente également une extrémité proximale (2c, 3c) située à l'opposée de l'extrémité distale (2a, 3a).

Par ailleurs, chaque barre d'ancrage (2, 3) présente, dans sa région distale, une pluralité, trois sur l'exemple représenté, de crans (2d, 3d) destinés à renforcer l'ancrage de chaque barre (2, 3) dans le fragment osseux et à limiter le déplacement de l'agrafe 1 dans le sens proximal une fois cette dernière insérée dans les fragments osseux à rapprocher.

L'agrafe 1 comporte en outre une barre de liaison 4, correspondant à la barre horizontale du U et reliant entre elles les extrémités proximales (2c, 3c) respectives des deux barres d'ancrage (2, 3). Comme il apparaît des figures, les deux barres d'ancrage (2, 3) et la barre de liaison 4 sont situées dans un même plan, le plan du U. Dans ce plan, les barres d'ancrage (2, 3) sont sensiblement parallèles entre elles.

La barre de liaison 4 comporte une ouverture longitudinale 5 la séparant en deux parois latérales (6, 7) opposées, reliées entre elles à leurs extrémités : ces extrémités correspondent aux points de jonctions (8, 9) de la barre de liaison 4 avec les barres d'ancrage (2,3).

En référence aux figures 3 et 4, les parois latérales (6, 7) sont déformables dans le plan P ; le plan P, représenté sur la figure 8, étant le plan contenant la barre de liaison 4 et perpendiculaire au plan contenant l'agrafe en forme de U. Comme il apparaît de ces figures, l'ouverture 5 est apte à adopter une configuration fermée (figure 3), dans laquelle les deux barres d'ancrage (2, 3) sont sensiblement parallèles entre elles et lesdites deux parois latérales (6, 7) sont rapprochées l'une de l'autre de telle sorte que la distance séparant les deux extrémités proximales (2c, 3c) des deux barres d'ancrage (2, 3) a une valeur L1, et au moins une configuration ouverte (figure 4), dans laquelle les régions centrales (6a, 7a) desdites deux parois latérales (6, 7) sont éloignées l'une de l'autre de part et d'autre de ladite ouverture 5, et la distance séparant les deux extrémités proximales (2c, 3c) des deux barres d'ancrage (2, 3) a une valeur L2 strictement inférieure à L1.

Comme il apparaît sur la figure 1, la face interne de la région centrale 6a de la paroi latérale 6 est munie d'un bourrelet 6b. Bien que non visible sur la figure, la face interne de la région centrale 7a de la paroi latérale 7 est munie d'un bourrelet 7b, identique au bourrelet de la paroi latérale opposée.

Par exemple, la barre de liaison 4 et les barres d'ancrage (2, 3) de l'agrafe 1 sont réalisées dans un matériau choisi parmi l'acier inoxydable, les alliages Chrome/Cobalt, l'acide polylactique, le polyétheréthercétone, le titane, et leurs mélanges.

L'agrafe comporte en outre une pièce de compression 20 sous la forme d'un jeton ayant la forme d'un cylindre de section transversale ovale : la pièce de compression 20 présente ainsi deux faces opposées de section ovale, une face proximale 20a et une face distale 20b, et une paroi périphérique 20c reliant les deux faces proximale 20a et distale 20b. La hauteur du cylindre étant beaucoup moins importante que les dimensions (largeur et longueur) des faces opposées, la pièce de compression est globalement comprise dans un plan A (plan du jeton) et comprend un axe B de rotation passant par le centre de chacune des faces de section ovale, et perpendiculaire à ce plan A de la pièce de compression. Dans l'exemple représenté sur les figures 1-4, la paroi périphérique 20c de la pièce de compression 20 est munie à mi-hauteur d'une rainure périphérique 22 : cette rainure périphérique 22 sépare ainsi la paroi périphérique 20c en une partie périphérique proximale 21a et une partie périphérique distale 21 b. Comme il apparaît des figures, les parties périphériques proximale 21 a et distale 21 b de la pièce de compression 20 présentent dans le plan de ladite pièce une section de forme globalement ovale. Ces parties périphériques proximale 21 a et distale 21 b de la pièce de compression 20 forment ensemble une portion dite portion active (21 a, 21 b) de la pièce de compression 20, qui, comme il apparaîtra de la description qui suit, est destinée à coopérer avec les faces internes des parois latérales (6, 7) afin de faire passer l'ouverture 5 de sa configuration fermée à une configuration ouverte.

Sur l'exemple représenté, la face proximale 20a de la pièce de compression 20 est munie d'un évidement 23 de section hexagonale : comme il apparaîtra dans la description qui suit, cet évidement 23 est apte à accueillir une clé de forme complémentaire dans le but d'imprimer à la pièce de compression un mouvement de rotation.

La pièce de compression 20 peut être réalisée dans un matériau choisi parmi l'acier inoxydable, les alliages Chrome/Cobalt, l'acide polylactique, le polyétheréthercétone, le titane, et leurs mélanges.

L'utilisation de l'agrafe 1 pour par exemple rapprocher deux fragments osseux va maintenant être décrite en référence aux figures 1-4 et 8.

L'agrafe 1 est fournie au chirurgien dans sa position assemblée telle que représentée aux figures 2 et 3, dans la configuration fermée de l'ouverture 5. Dans cette position, la pièce de compression 20 a été encliquetée au sein de l'ouverture 5 de telle sorte que sa portion active (21 a, 21 b) est dans le plan P de la barre de liaison 4, et les bourrelets (6b, 7b) des faces internes des parois latérales (6,7) sont engagés dans la rainure 22 de la paroi périphérique de la pièce de compression 20. Par ailleurs, dans cette configuration fermée de l'ouverture 5, le grand axe de la pièce de compression 20 est aligné sur l'axe longitudinal de la barre de liaison 4. La portion active (21 a, 21 b) de la pièce de compression 20, à savoir ses parties périphériques proximale 21 a et distale 21 b, sont en contact avec les faces internes des parois latérales (6, 7). Les barres d'ancrage (2, 3) sont sensiblement parallèles entre elles dans le plan de l'agrafe 1, autrement dit dans le plan du U formé par l'agrafe 1.

Comme il apparaît de la figure 3, dans la configuration fermée de l'ouverture 5, les parois latérales (6, 7) sont rapprochées l'une de l'autre de telle sorte que la distance séparant les deux extrémités proximales (2c, 3c) des deux barres d'ancrage (2, 3) a une valeur L1.

Pour procéder à une ostéosynthèse, ou au rapprochement de deux fragments osseux, par exemple d'un premier fragment osseux 30 et d'un deuxième fragment osseux 40 tels que montrés sur la figure 8, le chirurgien saisit, à l'aide d'un ancillaire approprié (non représenté), l'agrafe 1 dans la configuration fermée de l'ouverture 5 comme montré sur la figure 2, et il impacte l'agrafe 1 en insérant les deux barres d'ancrages (2, 3) par les parties pointues (2b, 3b) de leurs extrémités distales (2a, 3a) dans deux trous préalablement ménagés dans les fragments osseux (30, 40) à rapprocher.

Une fois l'agrafe 1 ainsi insérée, le chirurgien se munit d'un outil (non représenté) présentant une clé de forme complémentaire à l'évidement 23, et il imprime à la pièce de compression 20 une rotation autour de son axe B au sein de l'ouverture 5. Lors de cette rotation, la rainure 22 de la pièce de compression 20 et les bourrelets (6b, 7b) des faces internes des parois latérales (6, 7) servent de moyens de guidage, assurant que la rotation de la pièce de compression 20 se fait bien dans le plan P contenant la barre de liaison 4 et perpendiculaire au plan de l'agrafe 1. Par ailleurs, la portion active (21 a, 21 b) de la pièce de compression 20 étant de forme ovale et en contact avec les faces internes des parois latérales (6, 7), la rotation de la pièce de compression 20 provoque la déformation des parois latérales (6, 7) qui s'éloignent l'une de l'autre, comme montré sur la figure 4. L'éloignement des régions centrales (6a, 7a) des parois latérales provoque le rapprochement des extrémités proximales (2c, 3c) des barres d'ancrage (2, 3), et la distance séparant ces deux extrémités proximales (2c, 3c) a maintenant une valeur L2 strictement inférieure à L1, comme montré sur la figure 4.

Ainsi, la rotation de la pièce de compression 20, une fois les barres d'ancrage (2, 3) insérées dans les fragments osseux (30, 40) à rapprocher, permet d'ajuster, et en particulier, d'augmenter, la compression exercée par les deux barres d'ancrage (2, 3) sur lesdits fragments osseux (30, 40).

Sur la figure 8 est montrée l'agrafe 1 une fois insérée dans les fragments osseux (30, 40) et après rotation de la pièce de compression 20 comme décrite ci-dessus : la distance séparant les deux extrémités proximales (2c, 3c) des barres d'ancrage (2, 3) est donc L2, autrement dit, cette distance est inférieure à la distance L1 de l'étape d'impaction de l'agrafe 1. Les deux fragments osseux (30, 40) ont donc été rapprochés l'un contre l'autre par la rotation de la pièce de compression 20.

Dans l'exemple représenté à la figure 8, les deux barres d'ancrage (2, 3) sont également parallèles entre elles dans le plan de l'agrafe 1 lorsque l'ouverture 5 est en configuration ouverte, c'est-à-dire lorsque l'agrafe 1 est implantée dans les fragments osseux (30, 40) et que la pièce de compression 20 a subi la rotation décrite ci-dessus. En effet, dans cet exemple, la portion active (21 a, 21b) de la pièce de compression 20 agissant de façon homogène sur l'ensemble de la hauteur des parois latérales (6, 7), le rapprochement des extrémités proximales (2c, 3c) des barres d'ancrage (2, 3) s'accompagne d'un rapprochement parallèle des barres d'ancrages (2, 3) entre elles au sein des fragments osseux.

La pièce de compression 20 permet de fixer solidement les deux fragments osseux et d'éviter tout mouvement induisant un déplacement du montage ou une perte de comrpession.

Par ailleurs, la présence de la pièce de compression 20, qui reste solidaire de l'agrafe 1 une fois cette dernière implantée, permet d'empêcher la distension de l'agrafe et de maintenir l'ouverture 5 en configuration ouverte de façon permanente et ainsi de conserver la compression souhaitée sur les deux fragments osseux (30, 40) afin de les maintenir en position rapprochée jusqu'à ce que la fusion osseuse ait lieu.

De plus, la présence permanente de la pièce de compression 20, qui est plaquée sur les fragments osseux (30, 40), permet également d'empêcher le chevauchement de ces fragments osseux entre eux.

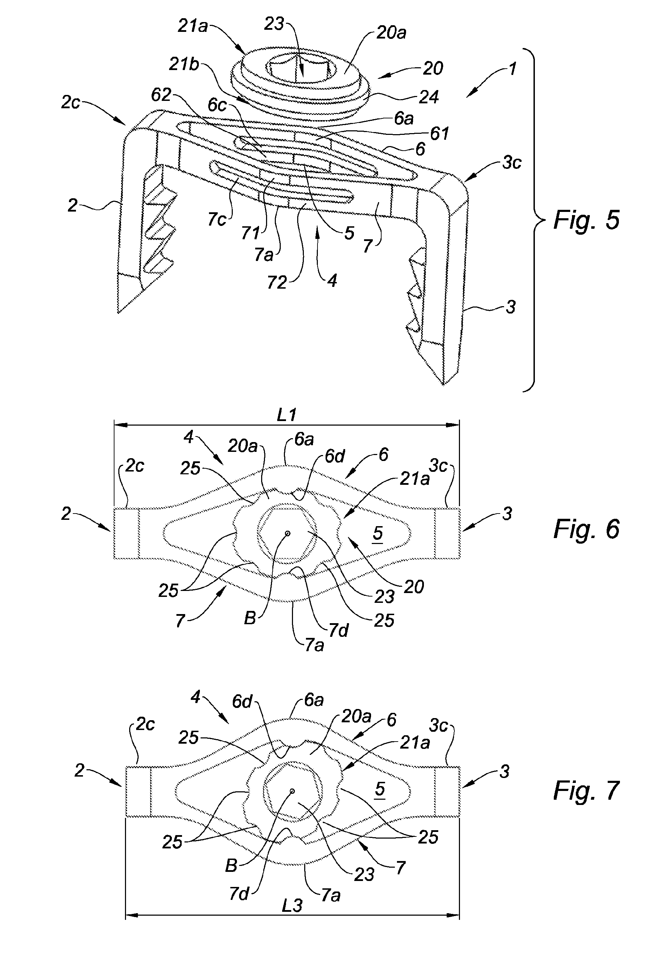

En référence à la figure 5, est montrée une variante de l'agrafe 1 de la figure 1, dans laquelle la rainure et les bourrelets des moyens de guidage de la rotation de la pièce de compression 20 ont été permutés entre la paroi périphérique 20c de la pièce de compression 20 et les faces internes des parois latérales (6, 7) de la barre de liaison 4, la rainure étant débouchante et formant une fente. Sur cette figure 5, les références désignant les mêmes éléments que dans les figures 1-4 ont été conservées.

La paroi périphérique 20c de la pièce de compression 20 est ainsi munie d'un bourrelet périphérique 24 et les faces internes des régions centrales (6a, 7a) des parois latérales (6, 7) sont munies chacune d'une fente longitudinale (6c, 7c). Le bourrelet périphérique 24 est situé à mi-hauteur de la paroi périphérique 20c et il détermine une partie périphérique proximale 21 a et une partie périphérique distale 21 b de la pièce de compression 20, qui ensemble forment la portion active (21 a, 21 b) de la pièce de compression 20.

Par ailleurs, la fente longitudinale 6c sépare la paroi latérale 6 en deux tronçons longitudinaux, un tronçon proximal 61 et un tronçon distal 62, reliés entre eux par leurs extrémités, chacun desdits tronçons proximal 61 et distal 62 étant déformable dans le plan P indépendamment de l'autre tronçon, comme il apparaîtra plus tard dans la description qui suit. De la même façon, la fente longitudinale 7c sépare la paroi latérale en deux tronçons longitudinaux, un tronçon proximal 71 et un tronçon distal 72, reliés entre eux par leurs extrémités, chacun desdits tronçons proximal 71 et distal 72 étant déformable dans le plan P indépendamment de l'autre tronçon.

Lorsque l'agrafe 1 de la figure 5 est sous sa forme assemblée et que la pièce de compression 20 est encliquetée dans l'ouverture 5, le bourrelet périphérique 24 est engagé dans les rainures (6c, 7c) des deux parois latérales (6, 7) et la portion active (21 a, 21 b) de la pièce de compression 20 est en contact avec les faces internes des parois latérales (6, 7) : le bourrelet périphérique 24 et les rainures (6c, 7c) agissent comme des moyens de guidage de la rotation de la pièce de compression 20 autour de son axe B et garantissent que cette rotation se fait bien dans le plan P. L'agrafe 1 de la figure 5 peut être utilisée pour procéder au rapprochement de deux éléments osseux comme décrit ci-dessus pour l'agrafe des figures 1-4.

En référence aux figures 6 et 7, est montrée une autre variante de l'agrafe 1 des figures 1-4 ou 5, comprenant en outre des moyens de blocage de la pièce de compression 20 à différents angles de rotation. Dans ces figures, les références désignant les mêmes éléments que dans les figures 1-4 ont été conservées.

Dans cette variante de l'agrafe 1 selon l'invention, la portion active (dont seule la partie périphérique proximale 21 a est visible sur les figures) de la pièce de compression 20 est munie d'une pluralité de reliefs, par exemple sous la forme de crans 25, répartis selon la périphérie de ladite portion active 21 a. Les faces internes des régions centrales (6a, 7a) des parois latérales (6, 7) sont chacune munies d'un relief complémentaire, par exemple sous la forme d'un ergot (6d, 7d) apte à s'enclencher dans un des crans 25 en vue de bloquer la rotation de la pièce de compression 20. Du fait de la forme ovale de la portion active 21a, il est ainsi possible de conférer à l'ouverture 5 plusieurs configurations ouvertes, avec une largeur d'ouverture pouvant varier : la largeur d'ouverture la plus grande étant atteinte lorsque le grand axe de la portion active 21 est perpendiculaire à l'axe longitudinal de la barre de liaison 4. Sur l'exemple représenté à la figure 7, l'ouverture est dans une configuration semi-ouverte, le grand axe de la portion active 21 a formant environ un angle de 45° avec l'axe longitudinal de la barre de liaison 4.

Ainsi, en reprenant les définitions données pour l'agrafe 1 des figures 1-4, la distance séparant les deux extrémités proximales (2c, 3c) sur la figure 7 a une valeur L3 comprise entre L1 et L2. Une telle forme de réalisation de l'agrafe 1 permet ainsi d'ajuster la force de la compression exercée par les barres d'ancrage (2, 3) sur les éléments osseux à rapprocher, entre une compression minimale, exercée lorsque l'ouverture 5 est dans sa configuration fermée, comme montrée sur la figure 6, et une compression maximale, exercée lorsque l'ouverture est dans sa configuration ouverte avec le grand axe de la portion active 21 a perpendiculaire à l'axe longitudinal de la barre de liaison 4.

Le blocage de la pièce de compression 20 à un certain angle de rotation peut être provisoire ou définitif selon la volonté du chirurgien. Ce dernier peut s'il le souhaite modifier l'angle de blocage au moyen d'un outil muni d'une clé comme décrit ci-dessus et débloquer la pièce de compression 20 pour la faire pivoter jusqu'à un angle de rotation différent.

En référence aux figures 9A à 10B sont montrées deux variantes d'une agrafe 1 similaire à celle de la figure 5, comprenant en outre des moyens de désalignement des barres d'ancrage, agencés pour provoquer alternativement l'écartement ou le rapprochement l'une de l'autre des régions distales respectives des deux barres d'ancrage, dans une configuration ouverte de l'ouverture. Sur ces figures, la partie en U de l'agrafe, à savoir les barres d'ancrage et la barre de liaison, diffère de celle de la figure 5 uniquement par l'absence des crans d'accroche (2d, 3d) des barres d'ancrage (2, 3), et les références désignant les mêmes éléments que dans la figure 5 ont été conservées.

En référence à la figure 9A est montrée une variante de la pièce de compression 20 de la figure 5 dans laquelle la partie de la pièce de compression 20 située proximalement au bourrelet périphérique 24 est dimensionnée de manière à ne pas être en contact avec les faces internes des parois latérales (6, 7) de la barre de liaison 4 lorsque la pièce de compression 20 est logée au sein de l'ouverture 5, et ce, quelle que soit la configuration de l'ouverture 5. En particulier, cette partie proximale de la pièce de compression 20, sous la forme d'un anneau 26 sur l'exemple représenté à la figure 9A, est dimensionnée de telle façon qu'elle ne coopère pas avec les tronçons proximaux (61, 71) des parois latérales (6, 7) (voir figure 5). Ainsi, l'anneau 26 présente un diamètre extérieur inférieur au diamètre de l'ouverture 5 et il ne fait donc pas partie de la portion active de la pièce de compression 20 : la portion active de la pièce de compression 20 de la figure 9A est limitée à la partie périphérique distale 21 b.

En conséquence, lorsque la pièce de compression 20 de la figure 9A est logée au sein de l'ouverture 5 de la partie en U de l'agrafe de la figure 5, et que la pièce de compression 20 est soumise à une rotation comme expliquée ci-dessus, seuls les tronçons distaux (62, 72) des parois latérales (6, 7) se déforment, comme montré sur la figure 9B : sur cette figure, pour des raisons de clarté, la pièce de compression 20 n'est pas représentée, mais la déformation montrée est celle causée par sa présence. Comme il apparaît de cette figure 9B, la déformation des tronçons distaux (62, 72) conjuguée à la non déformation ou très faible déformation des tronçons proximaux (61, 71) entraîne d'une part le rapprochement des extrémités proximales (2c, 3c) des barres d'ancrage comme déjà vu pour la figure 4, mais également le rapprochement des régions distales, en particulier des extrémités distales (2a, 3a), des deux barres d'ancrage (2, 3) dans une configuration ouverte de l'ouverture 5. Ainsi, cette forme de réalisation, telle que représentée aux figures 9A et 9B, permet d'augmenter encore la force de compression exercée par les deux barres d'ancrage (2, 3) sur deux éléments d'os à rapprocher.

En référence à la figure 10A est montrée une variante de la pièce de compression 20 de la figure 5 dans laquelle la partie de la pièce de compression 20 située distalement au bourrelet périphérique 24 est dimensionnée de manière à ne pas être en contact avec les faces internes des parois latérales (6, 7) de la barre de liaison 4 lorsque la pièce de compression 20 est logée au sein de l'ouverture 5, et ce, quelle que soit la configuration de l'ouverture 5. En particulier, cette partie distale de la pièce de compression 20, sous la forme d'un anneau 27 sur l'exemple représenté à la figure 10A, est dimensionnée de telle façon qu'elle ne coopère pas avec les tronçons distaux (62, 72) des parois latérales (6, 7) (voir figure 5). Ainsi, l'anneau 27 présente un diamètre extérieur inférieur au diamètre de l'ouverture 5 et il ne fait pas partie de la portion active de la pièce de compression 20 : la portion active de la pièce de compression 20 de la figure 10A est limitée à la partie périphérique proximale 21 a.

En conséquence, lorsque la pièce de compression 20 de la figure 10A est logée au sein de l'ouverture 5 de la partie en U de l'agrafe de la figure 5, et que la pièce de compression 20 est soumise à une rotation comme expliquée ci-dessus, seuls les tronçons proximaux (61, 71) des parois latérales (6, 7) se déforment, comme montré sur la figure 10B : sur cette figure, pour des raisons de clarté, la pièce de compression 20 n'est pas représentée, mais la déformation montrée est celle causée par sa présence. Comme il apparaît de cette figure 10B, la déformation des tronçons proximaux (61, 71) conjuguée à la non déformation ou très faible déformation des tronçons distaux (62, 72) entraîne d'une part le rapprochement des extrémités proximales (2c, 3c) des barres d'ancrage comme déjà vu pour la figure 4, mais également l'écartement des régions distales, en particulier des extrémités distales (2a, 3a), des deux barres d'ancrage (2, 3) dans une configuration ouverte de l'ouverture 5. Ainsi, cette forme de réalisation permet d'alléger la force de compression exercée par les deux barres d'ancrage (2, 3) sur deux éléments d'os à rapprocher.

L'agrafe selon l'invention permet de faire varier la compression exercée sur deux fragments osseux à rapprocher après impaction de l'agrafe, par exemple en augmentant ou en réduisant cette compression. Elle permet également de maintenir la force de compression choisie pendant le temps nécessaire à la consolidation osseuse.

## Revendications

1. Agrafe (1) pour ostéosynthèse en forme globale de U, comprenant :
- au moins deux barres d'ancrage (2, 3) correspondant aux deux barres verticales du U, chaque barre d'ancrage comprenant une extrémité distale (2a, 3a), destinée à être insérée dans un élément osseux (30, 40), et une extrémité proximale (2c, 3c), et
- au moins une barre de liaison (4), correspondant à la barre horizontale du U, reliant entre elles les extrémités proximales respectives des deux barres d'ancrage, ladite barre de liaison comprenant au moins une ouverture longitudinale (5) la séparant en deux parois latérales (6, 7) opposées reliées entre elles à leurs extrémités, déformables dans le plan P contenant la barre de liaison et perpendiculaire au plan contenant l'agrafe en forme de U, de telle sorte que l'ouverture est apte à adopter une configuration fermée, dans laquelle les deux barres d'ancrage sont sensiblement parallèles entre elles et lesdites deux parois latérales sont rapprochées l'une de l'autre de telle sorte que la distance séparant les deux extrémités proximales des deux barres d'ancrage a une valeur L1, et au moins une configuration ouverte, dans laquelle les régions centrales desdites deux parois latérales sont éloignées l'une de l'autre de part et d'autre de ladite ouverture, et la distance séparant les deux extrémités proximales des deux barres d'ancrage a une valeur L2 strictement inférieure à L1,
**caractérisée en ce que**
ladite agrafe comprend en outre une pièce de compression (20) présentant au moins une portion dite portion active (21 a, 21 b), dont la section dans un plan A de ladite pièce présente une forme globalement ovale, ladite pièce étant logée au sein de ladite ouverture avec son plan A confondu avec ledit plan P et au moins une partie de la face externe de sa portion active en contact avec au moins une partie des faces internes desdites deux parois latérales de la barre de liaison, ladite pièce étant apte à subir une rotation autour d'un axe B passant par le centre de ladite forme ovale de ladite portion active et perpendiculaire audit plan P, causant ainsi le passage de ladite ouverture de sa configuration fermée à au moins une dite configuration ouverte, et vice versa.

2. Agrafe (1) selon la revendication 1, **caractérisée en ce qu'**elle comprend des moyens de guidage (6b, 7b, 22) de la rotation de ladite pièce dans ledit plan P.

3. Agrafe (1) selon la revendication 2, **caractérisée en ce que** les moyens de guidage comprennent au moins une rainure (22 ; 6c, 7c) et un bourrelet (6b, 7b ; 24) aptes à s'engager l'un dans l'autre pour guider la rotation de ladite pièce dans ledit plan P, l'un de ladite rainure et dudit bourrelet étant situé sur les faces internes desdites parois latérales, l'autre de ladite rainure et dudit bourrelet étant situé sur ladite face externe de ladite portion active de ladite pièce.

4. Agrafe (1) selon l'une quelconque des revendications 1 à 3, **caractérisée en ce qu'**elle comprend des moyens de blocage (25, 6d, 7d) de ladite pièce à différents angles de rotation.

5. Agrafe (1) selon la revendication 4, **caractérisée en ce que** lesdits moyens de blocage comprennent une pluralité de reliefs (25) répartis selon la périphérie de ladite portion active, aptes à s'engager successivement dans un ou plusieurs reliefs complémentaires (6d, 7d) situés sur les faces internes desdites parois latérales, l'engagement d'un ou des reliefs dans le ou les reliefs complémentaires bloquant ladite pièce en rotation à un angle de rotation déterminé.

6. Agrafe (1) selon l'une quelconque des revendications précédentes, **caractérisée en ce qu'**elle comprend des moyens de désalignement (6c, 61, 62, 7c, 71, 72, 26, 27) desdites barres d'ancrage, agencés pour provoquer alternativement l'écartement ou le rapprochement l'une de l'autre des régions distales respectives desdites deux barres d'ancrage, dans une configuration ouverte de ladite ouverture.

7. Agrafe (1) selon la revendication précédente, **caractérisée en ce que** lesdits moyens de désalignement comprennent une fente longitudinale (6c, 7c) ménagée dans chaque paroi latérale et séparant ladite paroi en deux tronçons longitudinaux (61, 62, 71, 72), un tronçon proximal (61, 71) et un tronçon distal (62, 72), reliés entre eux par leurs extrémités, chacun desdits tronçons proximal et distal étant déformable dans le plan P indépendamment de l'autre tronçon.

8. Agrafe (1) selon la revendication précédente, **caractérisée en ce que**, lorsque ladite partie de ladite face externe de ladite portion active (21a) de ladite pièce est en contact avec les faces internes desdits tronçons proximaux (61, 71) desdites parois latérales seulement, lesdites régions distales desdites deux barres d'ancrage s'écartent l'une de l'autre quand ladite ouverture est dans une configuration ouverte.

9. Agrafe (1) selon la revendication 7, **caractérisée en ce que**, lorsque ladite partie de ladite face externe de ladite portion active (21b) de ladite pièce est en contact avec les faces internes desdits tronçons distaux (62, 72) desdites parois latérales seulement, lesdites régions distales desdites deux barres d'ancrage se rapprochent l'une de l'autre quand ladite ouverture est dans une configuration ouverte.

10. Agrafe (1) selon l'une quelconque des revendications précédentes, **caractérisée en ce que** les régions distales desdites deux barres d'ancrage sont munies de crans d'accroche (2d, 3d) destinés à renforcer l'ancrage desdites barres d'ancrage dans les éléments osseux (30, 40).

11. Agrafe (1) selon l'une quelconque des revendications précédentes, **caractérisée en ce que** la face proximale (20a) de ladite pièce comporte une surface (23) apte à coopérer avec un outil afin de transmettre un mouvement de rotation à ladite pièce.

## Patentansprüche

1. Klammer (1) für die Osteosynthese, im Allgemeinen im Form eines U, umfassend:
- mindestens zwei Verankerungsschenkel (2, 3), die den zwei vertikalen Schenkeln des U entsprechen, wobei jeder Verankerungsschenkel ein distales Ende (2a, 3a) umfasst, das ausgelegt ist, um in ein Knochenelement (30, 40) eingeführt zu werden, und ein proximales Ende (2c, 3c), und
- mindestens einen Verbindungsschenkel (4), der dem horizontalen Schenkel des U entspricht, der die jeweiligen proximalen Enden der beiden Verankerungsschenkel miteinander verbindet, wobei der Verbindungsschenkel mindestens eine längsgerichtete Öffnung (5) umfasst, die ihn in zwei gegenüberliegende Seitenwände (6, 7) trennt, die an ihren Enden miteinander verbunden sind, die in der Ebene P, die den Verbindungsschenkel enthält und senkrecht zur Ebene, die die Klammer in Form eines U enthält, verformbar ist, so dass die Öffnung dazu in der Lage ist, eine geschlossene Konfiguration anzunehmen, in der die zwei Verankerungsschenkel im Wesentlichen parallel zueinander sind, und die zwei Seitenwände derart einander angenähert sind, dass der Abstand, der die zwei proximalen Enden der zwei Verankerungsschenkel trennt, einen Wert L1 aufweist, und mindestens eine offene Konfiguration, in der die zentralen Bereiche der zwei Seitenwände voneinander auf beiden Seiten der Öffnung entfernt sind, und der Abstand, der die zwei proximalen Enden der zwei Verankerungsschenkel trennt, einen Wert L2 aufweist, der strikt kleiner als L1 ist,
**dadurch gekennzeichnet, dass**
die Klammer außerdem ein Druckstück (20) umfasst, das mindestens einen Abschnitt, genannt aktiven Abschnitt (21 a, 21 b), aufweist, dessen Schnitt in einer Ebene A des Stücks eine im Allgemeinen ovale Form aufweist, wobei das Stück innerhalb der Öffnung, mit seiner Ebene A in Übereinstimmung mit der Ebene P, aufgenommen ist, und mindestens einen Teil der Außenseite seines aktiven Abschnitts in Kontakt mit mindestens einem Teil der Innenseiten der zwei Seitenwände des Verbindungsschenkels, wobei das Stück dazu in der Lage ist, sich in Drehung um eine Achse B zu versetzen, die durch die Mitte der ovalen Form des aktiven Abschnitts und senkrecht zu der Ebene P verläuft, wodurch der Übergang der Öffnung aus ihrer geschlossenen Konfiguration in mindestens eine offene Position und umgekehrt hervorgerufen wird.

2. Klammer (1) nach Anspruch 1, **dadurch gekennzeichnet, dass** sie Mittel zur Führung (6b, 7b, 22) der Drehung des Stücks in der Ebene P umfasst.

3. Klammer (1) nach Anspruch 2, **dadurch gekennzeichnet, dass** die Mittel zur Führung mindestens eine Nut (22; 6c, 7c) und eine Wulst (6b, 7b; 24) umfassen, die dazu in der Lage sind, ineinander einzugreifen, um die Drehung des Stücks in der Ebene P zu führen, wobei eine der Nut und des Wulstes auf den Innenseiten der Seitenwände angeordnet ist und die andere der Nut und des Wulstes auf der Außenseite des aktiven Abschnitts des Teils angeordnet ist.

4. Klammer (1) nach einem der Ansprüche 1 bis 3, **dadurch gekennzeichnet, dass** sie Verriegelungsmittel (25, 6d, 7d) des Stücks bei verschiedenen Drehwinkeln umfasst.

5. Klammer (1) nach Anspruch 4, **dadurch gekennzeichnet, dass** die Verriegelungsmittel eine Vielzahl von Reliefs (25) umfassen, die entlang dem Umfang des aktiven Abschnitts verteilt sind, die dazu in der Lage sind, nacheinander in ein oder in mehrere komplementäre Reliefs (6d, 7d) einzugreifen, die auf den Innenseiten der Seitenwände angeordnet sind, wobei der Eingriff von einem oder der Relief(s) in das oder die komplementäre(n) Relief(s) das Stück in Drehung in einem bestimmten Drehwinkel verriegelt.

6. Klammer (1) nach einem der vorhergehenden Ansprüche, **dadurch gekennzeichnet, dass** sie Mittel zur Fehlausrichtung (6c, 61, 62, 7c, 71, 72, 26, 27) der Verankerungsschenkel umfasst, die angeordnet sind, um alternativ die Entfernung voneinander oder die Annäherung aneinander der entsprechenden distalen Bereiche bzw. der zwei Verankerungsschenkel in einer offenen Konfiguration der Öffnung hervorzurufen.

7. Klammer (1) nach dem vorhergehenden Anspruch, **dadurch gekennzeichnet, dass** die Mittel der Fehlausrichtung einen Längsschlitz (6c, 7c) umfassen, der in jeder Seitenwand gebildet ist und die Wand in zwei längsgerichtete Strecken (61, 62, 71, 72) trennt, eine proximale Strecke (61, 71) und eine distale Strecke (62, 72), die untereinander durch ihre Enden verbunden sind, wobei jede der proximalen und der distalen Strecke in der Ebene P unabhängig von der anderen Strecke verformbar ist.

8. Klammer (1) nach dem vorhergehenden Anspruch, **dadurch gekennzeichnet, dass**, wenn der Teil der Außenseite des aktiven Abschnitts (21 a) des Stücks nur mit den Innenseiten der proximalen Strecken (61, 71) der Seitenwände in Kontakt steht, sich die distalen Bereiche der zwei Verankerungsschenkel voneinander entfernen, wenn sich die Öffnung in einer offenen Konfiguration befindet.

9. Klammer (1) nach Anspruch 7, **dadurch gekennzeichnet, dass**, wenn der Teil der Außenseite des aktiven Abschnitts (21 b) des Stücks nur mit den Innenseiten der distalen Strecken (62, 72) der Seitenwände in Kontakt steht, sich die distalen Bereiche der zwei Verankerungsschenkel aneinander annähern, wenn sich die Öffnung in einer offenen Konfiguration befindet.

10. Klammer (1) nach einem der vorhergehenden Ansprüche, **dadurch gekennzeichnet, dass** die distalen Bereiche der zwei Verankerungsschenkel mit Befestigungsrasten (2d, 3d) versehen sind, die ausgelegt sind, um die Verankerung der Verankerungsschenkel in den Knochenelementen (30, 40) zu verstärken.

11. Klammer (1) nach einem der vorhergehenden Ansprüche, **dadurch gekennzeichnet, dass** die proximale Seite (20a) des Stücks eine Fläche (23) umfasst, die dazu in der Lage ist, mit einem Werkzeug zusammenzuarbeiten, um eine Drehbewegung auf das Stück zu übertragen.

## Claims

1. A clip (1) for a generally U-shaped osteosynthesis, comprising:
- at least two anchoring bars (2, 3) corresponding to the two vertical bars of the U-shape, each anchoring bar comprising a distal end (2a, 3a), intended to be inserted into a bone element (30, 40), and a proximal end (2c, 3c), and
- at least one linking bar (4), corresponding to the horizontal bar of the U-shape, linking together the respective proximal ends of both anchoring bars, said linking bar comprising at least a longitudinal opening (5) dividing it into two opposite side walls (6, 7) linked together at their ends, deformable in the plane P containing the linking bar and perpendicular to the plane containing the U-shaped clip, so that the opening is able to adopt a closed configuration, wherein both anchoring bars are substantially parallel to each other and said two side walls are moved closer to each other so that the distance between both proximal ends of both anchoring bars has a value L1, and at least an open configuration, wherein the central zones of said two side walls are moved away from each other on either side of said opening, and the distance between both proximal ends of both anchoring bars has a value L2 strictly lower than L1,
**characterized in that**
said clip further comprises a compression member (20) having at least a portion called active portion (21 a, 21 b), whose section in a plane A of said member has a generally oval shape, said member being housed into said opening with its plane A coincident with said plane P and at least part of the outer face of its active portion in contact with at least part of the inner faces of said two side walls of the linking bar, said member being able to be rotated around an axis B passing through the center of said oval shape of said active portion and perpendicular to said plane P, causing thus the passage of said opening from its closed configuration to at least one said open configuration, and vice versa.

2. The clip (1) according to claim 1, **characterized in that** it comprises guiding means (6b, 7b, 22) of the rotation of said member in said plane P.

3. The clip (1) according to claim 2, **characterized in that** the guiding means comprise at least a groove (22 ; 6c, 7c) and a bead (6b, 7b ; 24) able to be engaged into each other for guiding the rotation of said member in said plane P, one of said groove and of said bead being situated on the inner faces of said side walls, the other of said groove and of said bead being situated on the outer face of said active portion of said member.

4. The clip (1) according to any one of claims 1 to 3, **characterized in that** it comprises blocking means (25, 6d, 7d) of said member at different angles of rotation.

5. The clip (1) according to claim 4, **characterized in that** said blocking means comprise a plurality of reliefs (25) distributed according to the periphery of said active portion, able to be successively engaged into one or several complementary relief(s) (6d, 7d) situated on the inner faces of said side walls, the engagement of one relief or reliefs into the complementary relief(s) blocking said rotating member at a determined angle of rotation.

6. The clip (1) according to any one of the preceding claims, **characterized in that** it comprises misalignment means (6c, 61, 62, 7c, 71, 72, 26, 27) of said anchoring bars, arranged to alternatively cause the respective distal zones of said two anchoring bars to move closer to or away from each other, in an open configuration of said opening.

7. The clip (1) according to the preceding claim, **characterized in that** said misalignment means comprise a longitudinal slot (6c, 7c) arranged in each side wall and dividing said wall into two longitudinal segments (61, 62, 71, 72), a proximal segment (61, 71) and a distal segment (62, 72), linked together by their ends, each of said proximal and distal segments being deformable in the plane P independently from the other segment.

8. The clip (1) according to the preceding claim, **characterized in that**, when said part of said outer face of said active portion (21 a) of said member is in contact with the inner faces of said proximal segments (61, 71) of said side walls only, said distal zones of said two anchoring bars move away from each other when said opening is in an open configuration.

9. The clip (1) according to claim 7, **characterized in that**, when said part of said outer face of said active portion (21 b) of said member is in contact with the inner faces of said distal segments (62, 72) of said side walls only, said distal zones of said two anchoring bars move closer to each other when said opening is in an open configuration.

10. The clip (1) according to any one of the preceding claims, **characterized in that** the distal zones of said two anchoring bars are provided with grip notches (2d, 3d) intended to strengthen the anchoring of said anchoring bars in the bone elements (30, 40).

11. The clip (1) according to any one of the preceding claims, **characterized in that** the proximal face (20a) of said member includes a surface (23) able to cooperate with a tool in order to transmit a movement of rotation to said member.
